COMPLETE DOCUMENT

(19) Europäisches Patentamt
European Patent Office
Office européen des brevets

(11) Publication number: 0 108 403
A2

(12) EUROPEAN PATENT APPLICATION

(21) Application number: 83111010.1

(22) Date of filing: 04.11.83

(51) Int. Cl.³: C 07 D 311/82
G 01 N 33/58

(30) Priority: 08.11.82 US 440069

(43) Date of publication of application: 16.05.84 Bulletin 84/20

(84) Designated Contracting States: BE DE FR GB IT

(71) Applicant: ABBOTT LABORATORIES
14th Street and Sheridan Road
North Chicago, Illinois 60064(US)

(72) Inventor: Flentge, Charles Arthur
1003 Centurion Lane, No. 4
Vernon Hills Illinois(US)

(72) Inventor: Kirkemo, Curtis Lee
734 Chandler
Gurnee Illinois(US)

(74) Representative: Modiano, Guido et al,
MODIANO, JOSIF, PISANTY & STAUB Modiano & Associati Via Meravigli, 16
I-20123 Milan(IT)

(54) Substituted carboxyfluoresceins.

(57) This disclosure relates to a method and reagents for determining ligands in biological fluids such as serum, plasma, spinal fluid, amnionic fluid and urine. In particular, this disclosure relates to a fluorescence polarization immunoassay procedure and to a novel class of tracer compounds employed as reagents in such procedures.

EP 0 108 403 A2

Background Of The Invention

The present invention relates to a method and reagents for determining ligands in biological fluids such as serum, plasma, spinal fluid, amnionic fluid and urine. In particular, the present invention relates to a fluorescence polarization immunoassay procedure and to tracers employed as reagents in such procedures. The fluorescence polarization immunoassay techniques combine the specificity of an immunoassay with the speed and convenience of fluorescence polarization techniques to provide a means for determining the amount of a specific ligand present in a sample.

Competitive binding immunoassays for measuring ligands are based on the competition between a ligand in a test sample and a labeled reagent, referred to as a tracer, for a limited number of receptor binding sites on antibodies specific to the ligand and tracer. The concentration of ligand in the sample determines the amount of tracer that will specifically bind to an antibody. The amount of tracer-antibody conjugate produced may be quantitively measured and is inversely proportional to the quantity of ligand in the test sample.

In general, fluorescence polarization techniques are based on the principle that a fluorescent labeled compound when excited by linearly polarized light will emit fluorescence having a degree of polarization inversely related to its rate of rotation. Therefore, when a molecule such as a tracer-antibody conjugate having a fluorescent label is excited with linearly polarized light, the emitted light remains highly polarized because the fluorophore is constrained from rotating between the time light is absorbed and emitted. When a "free" tracer compound (i.e., unbound to an antibody) is excited by linearly polarized light, its rotation is much faster than the corresponding tracer-antibody conjugate and the molecules are more randomly oriented, therefore, the emitted light is depolarized. Thus, fluorescence polarization provides a quantitive means for measuring the amount of tracer-antibody conjugate produced in a competitive binding immunoassay.

Various fluorescent labeled compounds are known in the art. U. S. Patent No. 3,998,943 describes the preparation of a fluorescently labeled insulin derivative using fluorescein isothiocyanate (FITC) as the fluorescent label and a fluorescently labeled morphine derivative using 4-aminofluorescein hydrochloride as the fluorescent label. Carboxyfluorescein has also been used for analytical determinations. R. C. Chen, *Analytical Letters*, 10, 787 (1977) describes the use of carboxyfluorescein to indicate the activity of phospholipase. The carboxyfluorescein as described is encapsulated in lecithin liposomes, and it will fluoresce only when released by the hydrolysis of lecithin. U. S. application Serial Number 329,974, filed December 11, 1981, discloses a class of carboxyfluorescein derivatives useful as reagents in fluorescent polarization immunoassays wherein the carboxyfluorescein is directly bonded to a ligand-analog.

## Summary Of The Invention

The present invention encompasses a method for determining ligands in a sample comprising intermixing with said sample a biologically acceptable salt of a tracer of the formula:

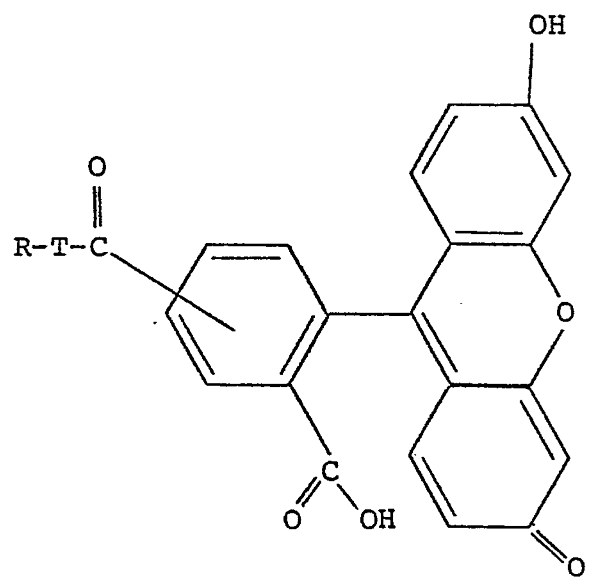

(I)

wherein T is a $-\overset{H}{\overset{|}{N}}-(CH_2)_n-\overset{H}{\overset{|}{N}}-$ group wherein n is an integer of from 1 to 8; and

wherein R is a ligand-analog having a reactive carbonyl group which is attached to the amino nitrogen of the groups represented by T wherein said ligand-analog has at least one common epitope with said ligand so as to be specifically recognizable by a common antibody;

and an antibody capable of specifically recognizing said ligand and said tracer; and then determining the amount of tracer antibody conjugate by fluorescence polarization techniques as a measure of the concentration of said ligand in the sample.

The invention further relates to certain novel tracers and biologically acceptable salts thereof, which are useful in reagents in the above-described method.

## Detailed Description Of The Invention

The term "ligand" as used herein refers to a molecule, in particular a low molecular weight hapten having a single reactive amino group, to which a binding protein, normally an antibody, can be obtained or formed. Such haptens are protein-free bodies, generally of low molecular weight that do not induce antibody formation when injected into an animal, but are reactive to antibodies. Antibodies to hapten are generally raised by first conjugating the haptens to a protein and injecting the conjugate product into an animal. The resulting antibodies are isolated by conventional antibody isolation techniques.

Ligands determinable by the method of the present invention vary over a wide molecular weight range. Although high molecular weight ligands may be determined, for best

results, it is generally preferable to employ the methods of the present invention to determine ligands of low molecular weight, generally in a range of 50 to 4000. It is more preferred to determine ligands having a molecular weight in a range of 100 to 2000.

Representative of ligands having a reactive carbonyl group determinable by the methods of the present invention include steroids such as esterone, estradiol, cortisol, testoestrone, progesterone, chenodeoxycholic acid, digoxin, cholic acid, digitoxin, deoxycholic acid, lithocholic acids and the ester and amide derivatives thereof; vitamins such as B-12, folic acid; thyroxine, triiodothyronine, histamine, serotonin, prostaglandins such as PGE, PGF, PGA; antiasthmatic drugs such as theophylline, antineoplastic drugs such as doxorubicin and methotrexate antiarrhythmic drugs such as disopyramide, lidocaine, procainamide, propranolol, quinidine, N-acetyl-procainamide; anticonvulsant drugs such as phenobarbital, phenytoin, primidone, valproic acid, carbamazepine and ethosuximide; antibiotics such as penicillins, cephalosporins and vancomycin, antiarthritic drugs such as salicylate; antidepressant drugs including tricyclics such as nortriptyline, amitriptyline, imipramine and desipramine; and the like as well as the metabolites thereof. Additional ligands that may be determined by the methods of the present invention include drugs of abuse such as morphine, heroin, hydromorphone, oxymorphone, metapon, codeine, hydrocodone, dihydrocodeine, dihydrohydroxy, codeinone, pholcodine, dextromethorphan, phenazocine and deonin and their metabolites.

The tracers of the present invention generally exist in an equilibrium between their acid and ionized states, and in the ionized state are effective in the method of the present invention. Therefore, the present invention

comprises the tracers in either the acid or ionized state and for convenience, the tracers of the present invention are structurally represented herein in their acid form. When the tracers of the present invention are present in their ionized state, the tracers exist in the form of biologically acceptable salts. As used herein, the term "biologically acceptable salts" refers to salts such as sodium, potassium, ammonium and the like which will enable the tracers of the present invention to exist in their ionized state when employed in the method of the present invention. Generally, the tracers of the present invention exist in solution as salts, the specific salt results from the buffer employed, i.e., in the presence of a sodium phosphate buffer, the tracers of the present invention will generally exist in their ionized state as a sodium salt.

The term ligand-analog as used herein refers to a mono or polyvalent radical a substantial proportion of which has the same spatial and polar organization as the ligand to define one or more determinant or epitopic sites capable of competing with the ligand for the binding sites of a receptor. A characteristic of such ligand-analog is that it possesses sufficient structural similarity to the ligand of interest so as to be recognized by the antibody for the ligand. For the most part, the ligand analog will have the same or substantially the same structure and charge distribution (spatial and polar organization) as the ligand of interest for a significant portion of the molecular surface. Since frequently, the linking site for a hapten will be the same in preparing the antigen for production of antibodies as used for linking to the ligand, the same portion of the ligand analog which provides the template for the antibody will be exposed by the ligand analog in the tracer.

In general, the group represented by R may be characterized as ligands or ligand analogs functionalized via a carboxyl, sulfonyl, or alkylhalo moiety which produces an amide or amine linkage with an amino nitrogen in the groups represented by T.

In accordance with the method of the present invention, a sample containing the ligand to be determined is intermixed with a biologically acceptable salt of a tracer of formula (I) and an antibody specific for the ligand and tracer. The ligand present in the sample and the tracer compete for limiting antibody sites resulting in the formation of ligand-antibody and tracer-antibody complexes. By maintaining constant the concentration of tracer and antibody, the ratio of ligand-antibody complex to tracer-antibody complex that is formed is directly proportional to the amount of ligand present in the sample. Therefore, upon exciting the mixture with polarized light and measuring the polarization of the fluorescence emitted by a tracer and a tracer-antibody complex, one is able to quantitatively determine the amount of ligand in the sample.

In theory, the fluorescence polarization of a tracer not complexed to an antibody is low, approaching zero. Upon complexing with a specific antibody, the tracer-antibody complex thus formed assumes the rotation of the antibody molecule which is slower than that of the relatively small tracer molecule, thereby increasing the polarization observed. Therefore, when a ligand competes with the tracer for antibody sites, the observed polarization of fluorescence of the tracer-antibody complex becomes a value somewhere

between that of the tracer and tracer-antibody complex. If a sample contains a high concentration of the ligand, the observed polarization value is closer to that of the free ligand, i.e., low. If the test sample contains a low concentration of the ligand, the polarization value is closer to that of the bound ligand, i.e., high. By sequentially exciting the reaction mixture of an immunoassay with vertically and then horizontally polarized light and analyzing only the vertical component of the emitted light, the polarization of fluorescence in the reaction mix may be accurately determined. The precise relationship between polarization and concentration of the ligand to be determined is established by measuring the polarization values of calibrators with known concentrations. The concentration of the ligand can be extrapolated from a standard curve prepared in this manner.

The pH at which the method of the present invention is practiced must be sufficient to allow the tracers of formula (I) to exist in their ionized state. The pH may range from about 3 to 12, more usually in the range of from about 5 to 10, most preferably from about 6 to 9. Various buffers may be used to achieve and maintain the pH during the assay procedure. Representative buffers include borate, phosphate, carbonate, tris, barbital and the like. The particular buffer employed is not critical to the present invention, but in an individual assay, a specific buffer may be preferred in view of the antibody employed and ligand to be determined. The cation portion of the buffer will generally determine the cation portion of the tracer salt in solution.

The methods of the present invention are practiced at moderate temperatures and preferably at a constant temperature. The temperature will normally range from about 0° to 50° C, more usually from about 15° to 40° C.

The concentration of ligand which may be assayed will generally vary from about $10^{-2}$ to $10^{-13}$M, more usually from about $10^{-4}$ to $10^{-10}$M. High concentrations of ligand may be assayed upon dilution of the original sample.

In addition to the concentration range of ligand of interest, considerations such as whether the assay is qualitative, semiquantitative or quantitative, the equipment employed, and the characteristics of the tracer and antibody will normally determine the concentration of the tracer and antibody to be employed. While the concentration of ligand in the sample will determine the range of concentration of the other reagents, i.e., tracer and antibody, normally to optimize the sensitivity of the assay, individual reagent concentrations will be determined empirically. Concentrations of the tracer and antibody are readily ascertained by one of ordinary skill in the art.

The preferred tracers of the present invention are characterized as derivatives of 5-carboxyfluorescein or 6-carboxyfluorescein or mixtures thereof and are represented by the formulas:

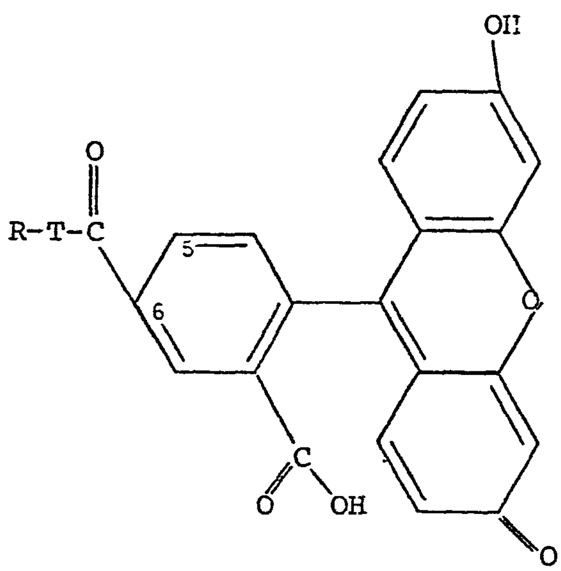

(V)

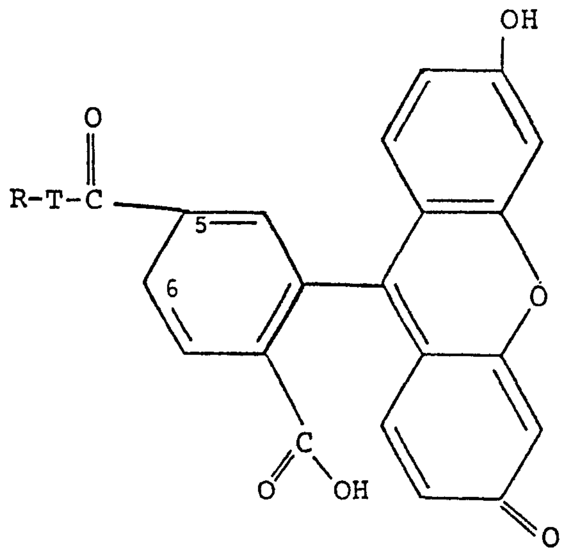

(VI)

The following illustrative, nonlimiting examples will serve to further demonstrate to those skilled in the art the manner in which specific tracers within the scope of this invention may be prepared. The symbol [*CF*] appearing in the structural formulas illustrating the compounds prepared in the following examples, represents a moiety of the formula:

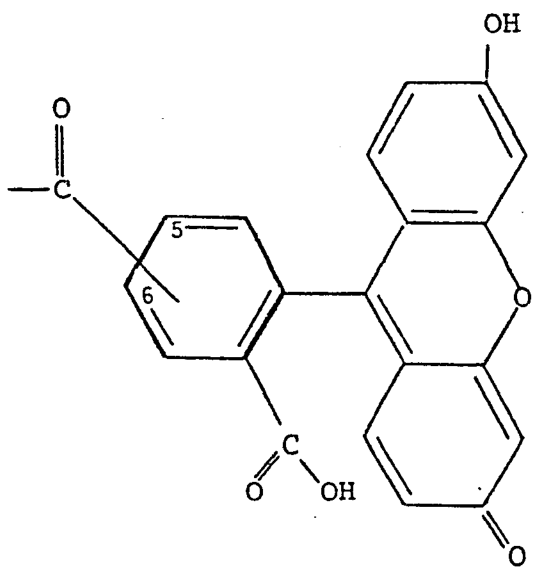

(VII)

wherein the carbonyl carbon is attached to the 5 or 6 position depending on whether the starting material employed in the Example is 5-carboxyfluorescein, 6-carboxyfluorescein or a mixture thereof.

## EXAMPLE 1

### PREPARATION OF N-HYDROXYSUCCINIMIDE ACTIVE ESTER OF CARBOXYFLUORESCEIN

To 83 mg (0.22 mmol) of 6-carboxyfluorescein dissolved in 2 ml of dimethylformamide was added 28 mg (0.24 mmol) of N-hydroxysuccinimide and 55 mg (0.27 mmol) of N,N'-dicyclohexylcarbodiimide. The reaction mixture was stirred at 0° C under argon atmosphere for one hour and then maintained at 4° C for 16 hours to yield a N-hydroxysuccinimide active ester of carboxyfluorescein having the formula:

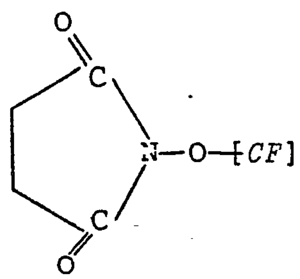

EXAMPLE 2

D-thyroxine sodium salt (100 mg, 0.00012 mole) is added with constant stirring to a solution of di-t-butyl dicarbonate (27 mg, 0.00012 mole) in 1 ml of dimethylformamide. After stirring two hours at room temperature the solvent is removed under high vacuum. The residue is dissolved in methanol and 1N HCl is added dropwise until a precipitate is formed. The solvent is decanted and the residue is dried under vacuum to yield 90 mg (82% yield) of N-t-butoxycarbonyl D-thyroxine having the formula:

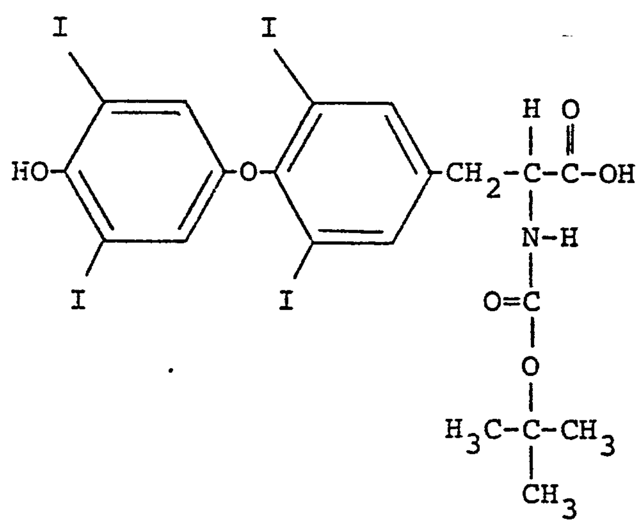

To a portion of the N-t-butoxycarbonyl D-thyroxine (51 mg, 0.00006 mole) dissolved in 1 ml of dimethylformamide is added N-hydroxysuccinimide (7 mg, 0.00006 mole) and N,N'-dicyclohexylcarbodiimide (15 mg, 0.00006 mole). The reaction mixture is stirred at room temperature for three hours after which time the reaction mixture is filtered into a solution of 1,3-diaminopropane (50 μℓ, 0.0003 mole) in 1 ml dimethylformamide with constant stirring. After stirring for fifteen minutes at room temperature, the solvent is removed and the residue is purified on a Whatman C18 reversed phase prep TLC plate eluting with a 80/19/1 mixture of methanol-water-acetic acid to yield 33 mg, (61% yield) of N-t-butoxycarbonyl D-thyroxine propylene diamine having the formula:

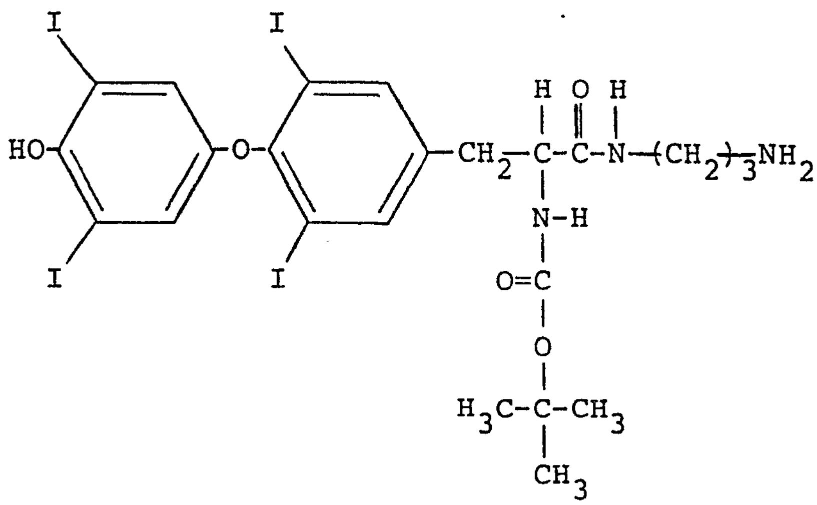

To a portion of the N-t-butoxycarbonyl D-thyroxine propylenediamine (10 mg, 0.00001 mole) dissolved in 1 ml of dimethylformamide is added N-hydroxysuccinimide active ester of a mixture of 5- and 6-carboxyfluorescein (5 mg, 0.00001 mole). The reaction mixture is stirred for thirty minutes at room temperature and the solvent is removed under vacuum. The residue is purified using a silica gel prep TLC plate eluting with a 9:1 mixture of methylene chloride:methanol to yield a mixture of N-t-butoxycarbonyl D-thyroxine propylenediamine carboxyfluorescein having the general formula:

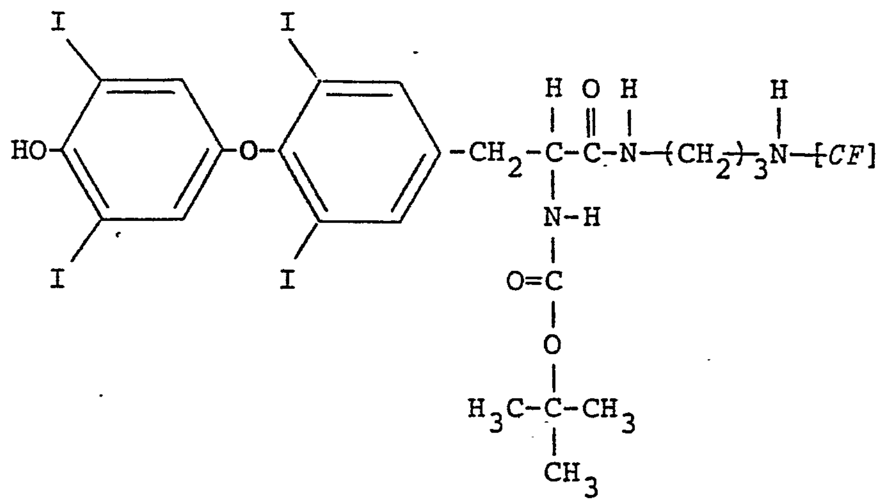

The mixture of N-t-butoxycarbonyl D-thyroxine propylenediamine carboxyfluoresceins is suspended in methylene chloride (1 ml) and cooled to 0° C. Trifluoroacetic acid (1 ml) is added and the reaction is stirred for one hour at 0° C. The solvent is removed under vacuum and purification of the product isomers is performed on a Whatman C18 reversed phase prep TLC plate eluting with 80/19/1 mixture of methanol-water-acetic acid to yield D-thyroxine propylenediamine-5-carboxyfluorescein and D-thyroxine propylenediamine 6-carboxyfluorescein trifluoroacetic acid salts having the general formula:

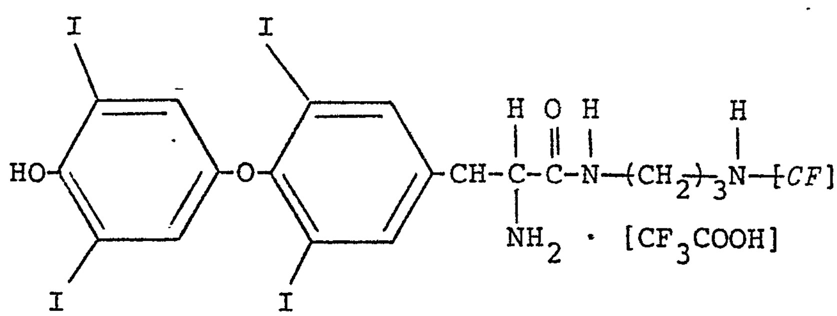

Compound 1

0108403

## EXAMPLE 3

To N-t-butoxycarbonyl D-thyroxine (51 mg:0.00006 mol) dissolved in 1 ml of dimethylformamide is added N-hydroxysuccinimide (7 mg: 0.00006 mol) and N,N'-dicyclohexylcarbodiimide (15 mg:0.0006 mol). The reaction mixture is stirred at room temperature for three hours after which time the reaction mixture is filtered into a solution of 1,4-diamino-butane (60 μℓ:0.0003 mol) in 1 ml dimethylformamide with constant stirring. After stirring for fifteen minutes at room temperature, the solvent is removed and the residue is purified on a Whatman C18 reverse phase prep thin-layer chromatography plate eluting with a 80:19:1 mixture of methanol:water:acetic acid to yield 31 mg, (56% yield) of N-t-butoxycarbonyl-D-thyroxinebutylenediamine having the formula:

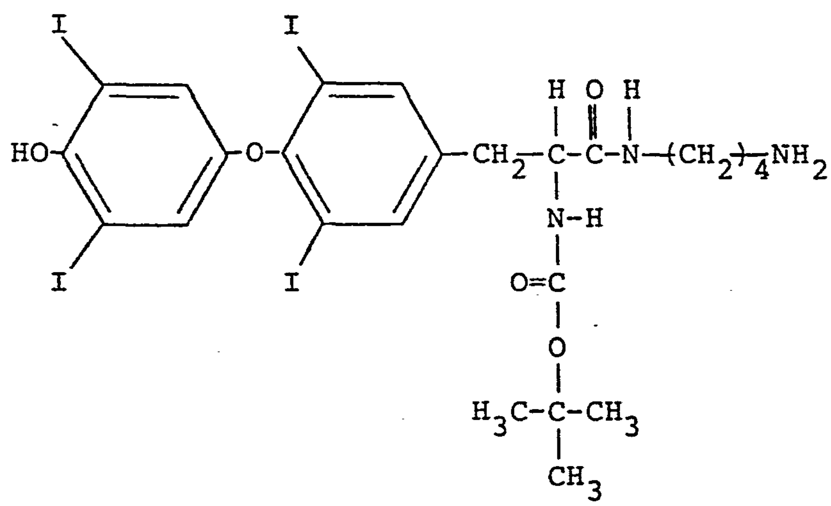

To a portion of the N-t-butoxycarbonyl D-thyroxine butylenediamine (10 mg:0.00001 mol) dissolved in 1 ml of dimethylformamide is added an N-hydroxysuccinimide active ester of a mixture of 5- and 6-carboxyfluorescein (5 mg:0.00001 mol). The reaction mixture is stirred for thirty minutes at room temperature the solvent is removed under vacuum. The residue is purified using a silica gel prep thin-layer chromatography plate eluting with a 9:1 mixture of methylene chloride:methanol to yield a mixture of N-t-butoxycarbonyl D-thyroxinebutylenediamine carboxyfluorescein having the general formula:

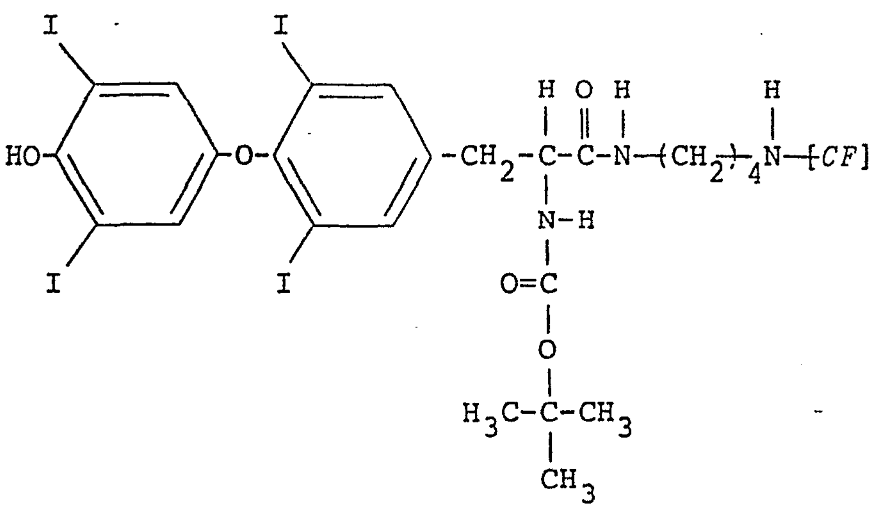

The mixture of N-t-butoxycarbonyl D-thyroxine butylenediamine carboxyfluorescein is suspended in methylene chloride (1 ml) and cooled to 0° C. Trifluoroacetic acid (1 ml) is added and the reaction is stirred for one hour at 0° C. The solvent is removed under vacuum and the residue is purified utilizing reversed phase prep thin-layer chromatography plate eluting with an 80:19:1 mixture of methanol:water:acetic acid to yield D-thyroxinebutylenediamine 5-carboxyfluorescein and D-thyroxinebutylenediamine 6-carboxyfluorescein trifluoroacetic acid salts having the general formula:

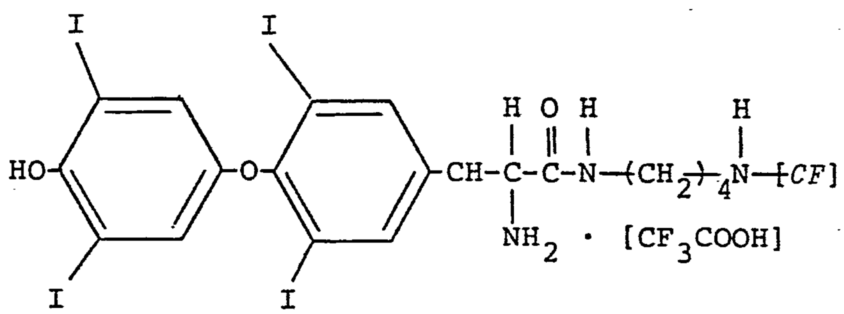

Compound 2

0108403

EXAMPLE 4

To N-t-butoxycarbonyl D-thyroxine (100 mg;0.114 mmol) dissolved in 1 ml of dimethylformamide is added N-hydroxysuccinimide (30 mg:0.2 mmol) and N,N'-dicyclohexylcarbodiimide (25 mg:0.121 mmol). The reaction mixture is stirred at room temperature for 2.5 hours after which time the reaction mixture is filtered into a solution of ethylenediamine (100 μℓ:1.5 mmol) in 1 ml dimethylformamide with constant stirring. A residue which had formed is dissolved in dimethylformamide and the resulting solvent is evaporated and the residue is purified on a reverse phase prep thin-layer chromatography plate eluting with a mixture of methanol:water:acetic acid to yield 54 mg, (52% yield) of N-t-butoxycarbonyl D-thyroxine ethylenediamine having the formula:

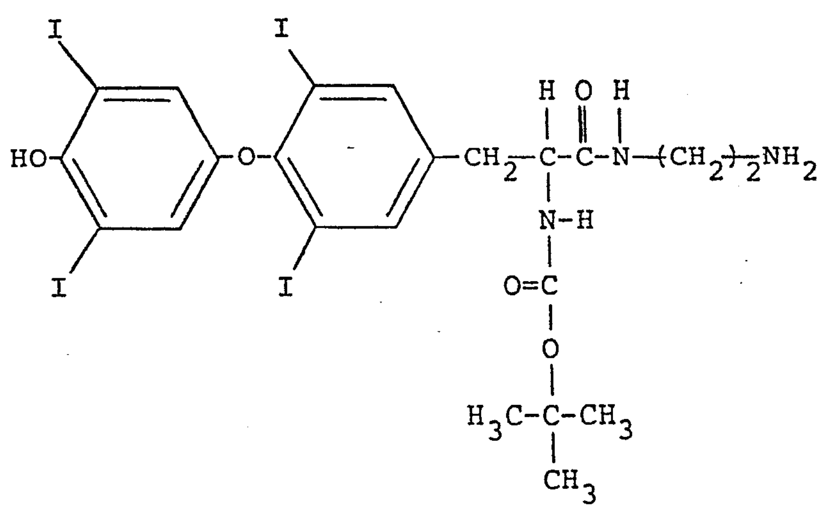

To a portion of the N-t-butoxycarbonyl D-thyroxine ethylenediamine (23 mg:0.025 mmol) dissolved in 1 ml of dimethylformamide is added succinimide active ester of 6-carboxyfluorescein (13 mg:0.025 mmol). The reaction mixture is stirred for one hour at room temperature the solvent is removed under vacuum. The residue is purified using a silica gel prep thin-layer chromatography plate eluting with a mixture of methylene chloride:methanol:acetic acid to yield a N-t-butoxycarbonyl D-thyroxine ethylenediamine-6-carboxyfluorescein having the formula:

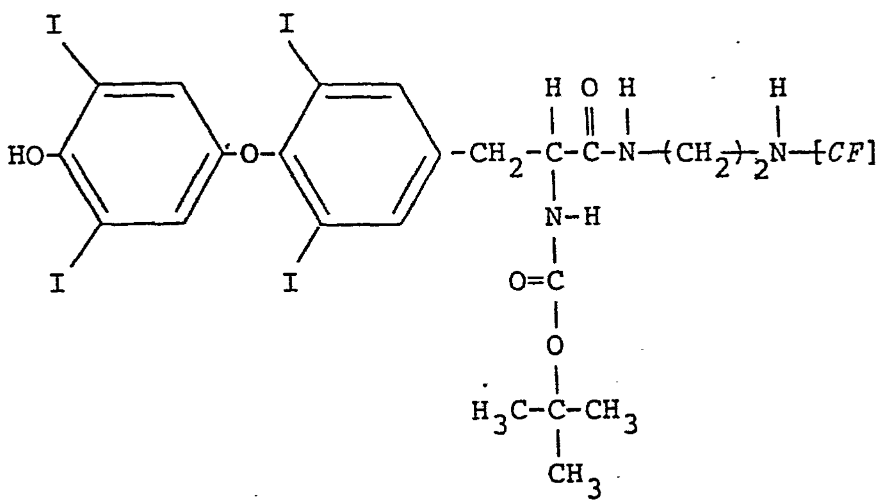

A portion of the N-t-butoxycarbonyl D-thyroxine ethylenediamine-6-carboxyfluorescein is suspended in 0.6 ml of a 1:1 mixture of methylene chloride:trifluoroacetic acid. The reaction is stirred for twenty minutes at 0° C and the solvent is removed under vacuum and the residue is purified utilizing a reversed phase prep thin-layer chromatography plate eluting with a mixture of methanol:water:acetic acid to yield D-thyroxine ethylenediamine-6-carboxyfluorescein trifluoroacetic acid salt having the formula:

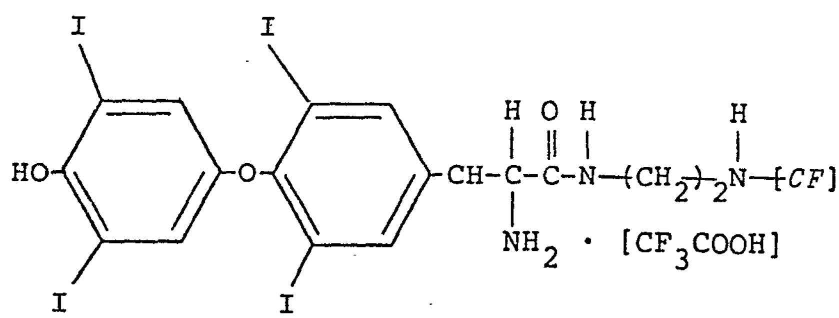

Compound 3

As previously mentioned, the tracers of the present invention are effective reagents for use in fluorescence polarization immunoassays. The following Examples illustrate the suitability of tracers of the present invention in immunoassays employing fluorescence polarization techniques. Such assays are conducted in accordance with the following general procedure:

1) A measured volume of standard or test serum is delivered into a test tube and diluted with buffer;

2) A known concentration of a tracer of the present invention optionally containing a surfactant is then added to each tube;

3) A known concentration of antisera is added to the tubes;

4) The reaction mixture is incubated at room temperature; and

5) The amount of tracer bound to antibody is measured by fluorescence polarization techniques as a measure of the amount of ligand in the sample.

## EXAMPLE 5

## THYROXINE ASSAY

A. Materials required:

1) BGG buffer consisting of 0.1M sodium phosphate, pH 7.5, containing bovine gammaglobulin, 0.01% and sodium azide, 0.01%.

2) Tracer, consisting of Compound 3 prepared in Example 4 at a concentration of approximately 60nM in BGG buffer.

3) Antiserum, consisting of sheep antiserum raised against thyroxine, diluted appropriately in BGG buffer.

4) Samples of human serum or other biological fluid containing thyroxine.

5. Serum denaturing reagent--8M urea, 3% sodium dodecyl sulfate, 1% dithioerythritol, 50mM ascorbic acid, 2mM sodium editate in water.

6. Cuvettes, 10 X 75 mm glass culture tubes used as cuvettes.

7) Fluorometer capable of measuring fluorescence polarization with a precision of ± 0.001 units.

B. Assay protocol:

1. To 50 μℓ of serum denaturing reagent in a test tube was added 50 μℓ of a standard or unknown sample. The tubes containing the sample were capped and vortexed.

2. A small volume of sample (20 microliters) is placed in each cuvette by pipetting 29 μℓ of denatured sample and diluting to 500 μℓ BGG buffer containing 25 ml pretreatment reagent and 25 ml antiserum in a dilution vessel. Next 175 μℓ of diluted sample is pipetted into the cuvette followed by 805 μℓ BGG buffer. Serum background fluorescence is read at this point.

3. Tracer is added by pipetting .75 μℓ diluted sample and 25 μℓ tracer and 780 μℓ BGG buffer into the cuvette.

4. The contents of all cuvettes are well mixed and allowed to incubate for 4 minutes at 35° C temperature.

5. The fluorescence polarization is read on a fluorometer corrected for serum fluorescence background and a standard curve constructed to determine unknowns.

C. The results of a series of serum standards containing thyroxine at concentrations between 0 and 24 μg/dL are presented below. Each concentration was assayed in duplicate and averaged.

| Concentration of Thyroxine (μg/dL) | Polarization |
|---|---|
| 0 | .217 |
| 3 | .211 |
| 6 | .198 |
| 12 | .173 |
| 18 | .159 |
| 24 | .154 |

The polarization of fluorescence is seen to decrease in a regular manner as the thyroxine concentration

increases, allowing construction of a standard curve. Unknown specimens treated in an identical manner can be quantitated by reference to the standard curve, thereby illustrating the utility of thyroxine carboxyfluorescein for the measurement of thyroxine derivatives.

As evident from the above results, the tracers of the present invention are effective reagents in fluorescence polarization immunoassays. In addition to the properties mentioned above, the tracers of the present invention possess a high degree of thermal stability, a high degree of bound polarization, high quantum yields and are relatively easy to produce and purify.

In addition to being useful as reagents in a fluorescence polarization immunoassay, the thyroxine carboxyfluorescein derivatives of the present invention may be useful as tracers in a fluorescence polarization assay to determine unsaturated thyroxine binding protein sites ("T uptake") in accordance with the procedure of the following Example:

EXAMPLE 6

A. Reagents

1. Pretreatment Solution - A solution containing 0.15% sodium dodecyl sulfate 0.564 M triethylenediamine (DABCO), and 0.1% sodium azide in 0.1M sodium phosphate buffer (pH. 7.25).

2. $T_4$ - Fluorescein Tracer - Consisting of Compound 1 prepared in Example 2 is employed at a concentration of 2.4 x $10^{-7}$M in a buffered medium containing .005% sodium dodecyl sulfate, 0.1% bovine gamma globulin, and 0.1% sodium azide in 0.1M sodium phosphate buffer.

3. T Uptake Calibrators - Sheep anti-T antisera in a 4% human serum matrix having the following uptake values: 0, 0.5, 1.0, 1.5, 2.0, and 2.5. An uptake value of 1.0 is equivalent to the T uptake of normal serum.

4. Diluent buffer: 0.1M sodium phosphate containing 0.1% bovine gamma globulin and 0.1% sodium azide.

All polarized fluorescence measurements were made using a polarization spectrofluorimeter (Abbott $TD_x$™ Fluorescence Polarization Analyzer.

B. Assay Protocol

1. To 1 μℓ aliquot of an unknown sample is added 25 μℓ of the pretreatment solution and the resulting mixture is diluted to 1 ml with diluent buffer. The resultant assay solution is mixed and the polarized fluorescence background is measured.

2. To the assay solution in Step 1. is added a second 1 μℓ aliquot of the unknown sample, 25 μℓ of the pretreatment solution 25 μℓ of $T_4$ fluorescein tracer, and the buffer to a final volume of 2 ml. The resultant solution is mixed and the polarized fluorescence is measured.

3. The fluorescence polarization due to tracer binding is obtained by subtracting the polarized fluorescence intensities of the background from the final polarized fluorescence intensities of the mixture.

4. The polarization values obtained are proportional to the T uptake of each sample.

5. The fluorescence polarization for a sample is cmopared to a standard curve prepared using calibrators of known T uptake values to indicate the T uptake value.

Although this invention has been described with respect to specific modifications, the details thereof are not to be construed as limitations, for it will be apparent that various equivalents, changes and modifications may be resorted to without departing from the spirit and scope thereof and it is understood that such equivalent embodiments are intended to be included therein.

## CLAIMS

1. A compound of the formula:

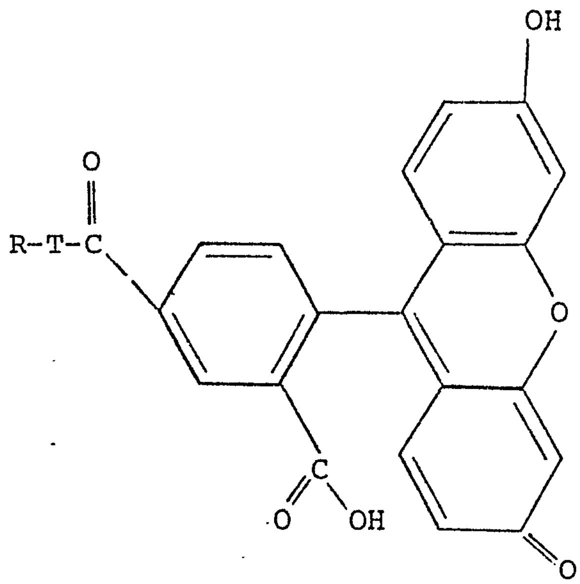

or

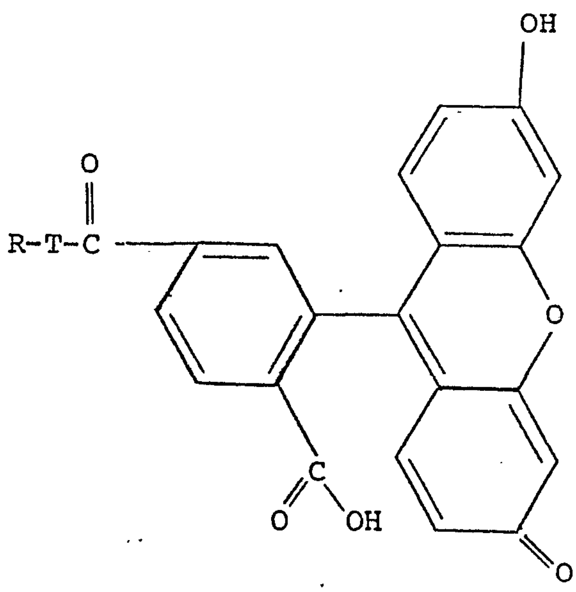

wherein T is a $-\overset{H}{\overset{|}{N}}-(CH_2)_n\overset{H}{\overset{|}{N}}$-group wherein n is an integer of from 1 to 8;

and R is a ligand-analog having at least one common epitope with a ligand so as to be specifically recognizable by a common antibody;

and biologically acceptable salts thereof.

2. A compound according to Claim 1 wherein R is a thyroxine analog.

3. A compound according to Claim 2 wherein R is

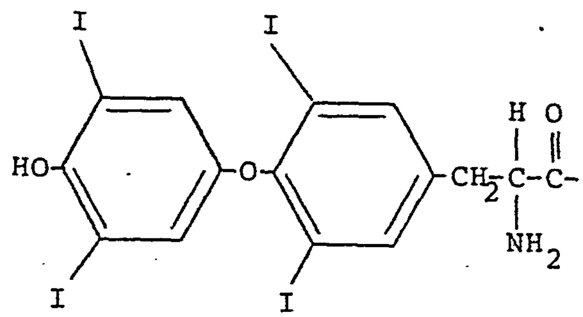

4. A compound according to Claim 3 wherein n is an integer of from 2 to 4.

5. A compound according to Claim 4 wherein n is 3.

6. A method for determining ligands in a sample comprising intermixing with said sample a tracer of the formula:

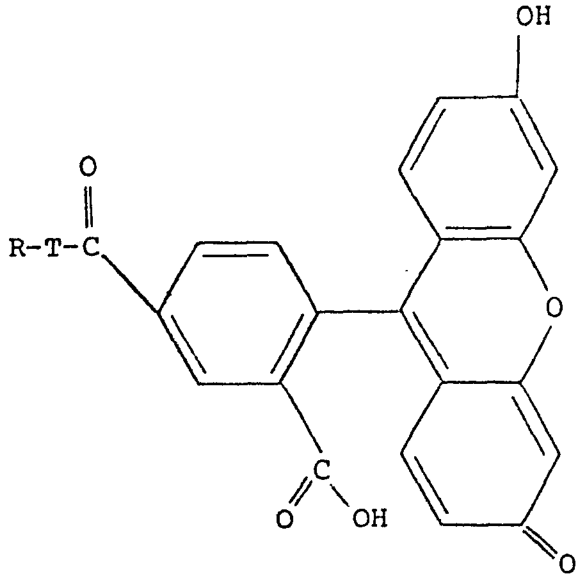

or

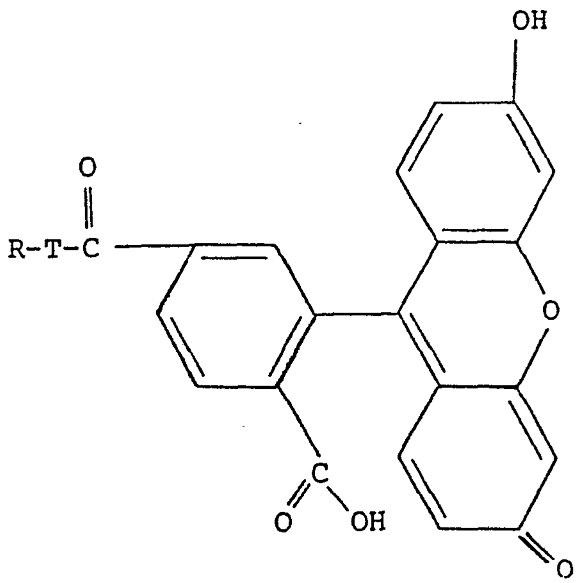

wherein T is a $-\overset{\overset{H}{|}}{N}-(CH_2)_n-\overset{\overset{H}{|}}{N}-$group wherein n is an integer of from 1 to 8;

and R is a ligand-analog having at least one common epitope with a ligand so as to be specifically recognizable by a common antibody;

and biologically acceptable salts thereof; and an antibody capable of specifically recognizing said ligand and said tracer; and then determining the amount of tracer bound to antibody by fluorescence polarization techniques as a measure of the amount of ligand in the sample.

7. A method according to Claim 6 wherein R" has a molecular weight within a range of 50 to 4000.

8. A method according to Claim 7 wherein R is a thyroxine analog.

9. A method according to Claim 8 wherein R is

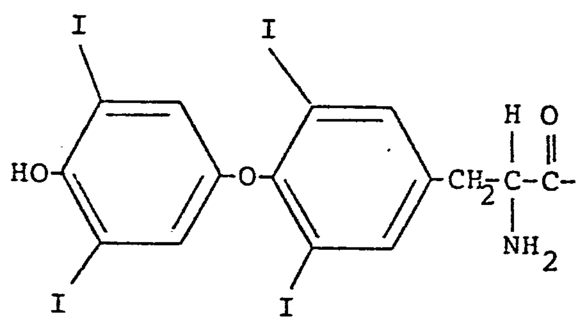

10. A method according to Claim 6 or 9 wherein n is an integer of from 2 to 4.

11. A method according to Claim 10 wherein n is 3.